# EUROPEAN PATENT APPLICATION

(11) **EP 0 726 063 A1**
(43) Date of publication of application: **14.08.1996**
(21) Application number: 96200310.9
(22) Date of filing: 12.02.1996
(51) Int. Cl.: A61B 17/17

(54) **Orthopaedic milling guide with cutter lockout**

(30) Priority: 13.02.1995 US 387510
(71) Applicant: Bristol-Myers Squibb Company, New York, N.Y. 10154 (US)
(72) Inventor: Stalcup, Gregory C., Columbia City, IN 46725 (US); Dietzel, Steven E., Peru, IN 46970 (US)
(74) Representative: Mays, Julie

(57) **Abstract**

The invention is directed to a milling guide for use in orthopaedic surgery for guiding one of a plurality of milling cutters. A first cutter has a first diameter, and a second cutter has a second diameter which is larger than the first diameter. The milling guide includes a guide body having a slot. The slot is configured to slidingly receive the first cutter. The guide body also includes structure associated with the slot for receiving the first cutter in the slot and preventing reception of the second cutter in the slot.

## Description

### BACKGROUND OF THE INVENTION

### 1. Field of the invention.

The present invention relates to instrumentation used in orthopaedic surgery, and, more particularly, to instrumentation used to prepare bone for receiving a prosthesis.

### 2. Description of the related art.

In an orthopaedic surgery to replace part or all of a patient's joint with a prosthetic implant, a portion of the implant receiving bone is prepared to closely match the mating surfaces of the implant. During an orthopaedic surgery to replace a knee joint, the distal end of the femur is prepared to accommodate a femoral knee component and the proximal end of the tibia is prepared to accommodate a tibial component.

Depending on the type of femoral implant to be accommodated by the femur, a notch may be required in the distal end of the resected femoral bone. Typically, such a notch is required to accommodate implants referred to as constrained condylar knees and posterior stabilized knees. With these type implants, the posterior and anterior cruciate ligaments of the knee are not functioning properly or have been removed as determined by the surgeon. The implant is therefore required to replace the functions of the ligaments. It is common for the femoral implant to include some type of protrusion which extends upwardly from the tibial plate and into the femur. It is thus necessary to form a notch in the distal end of the femur to accommodate the protrusion. If the notch is formed using a milling procedure (as opposed to a sawing procedure), then a milling cutter having a diameter of three-quarter (3/4) inch is typically used.

A problem with using a standard 3/4 inch milling cutter to form the notch in the end of the femur is that when the femur to be cut is relatively small in size, an excess amount of bone may be removed therefrom. For example, the femoral implant is sized according to the physical dimensions of the distal femur. A size "A" and "B" femoral implant corresponds to a femur which is relatively small. To form the notch in such a bone, it is desirable to use a smaller milling cutter, such as a 1/2 inch milling cutter, to avoid removing an excess amount of bone.

A problem with conventional milling guides is that a large milling cutter may be used when cutting the notch in the femur, thereby resulting in an excess amount of bone being removed from the end of the femur.

What is needed in the art is a size "A" and/or "B" milling guide which prevents the use of a relatively large, e.g., 3/4 inch, milling cutter when forming a notch in the end of a femur.

### SUMMARY OF THE INVENTION

The present invention provides a milling guide for use with a small femur which allows the use of a relatively small, e.g., 1/2 inch, milling cutter, and prevents the use of a relatively large, e.g., 3/4 inch, milling cutter.

The invention comprises, in one form thereof, a milling guide for use in orthopaedic surgery for guiding one of a plurality of milling cutters. A first cutter has a first diameter, and a second cutter has a second diameter which is larger than the first diameter. The milling guide includes a guide body having a slot. The slot is configured to slidingly receive the first cutter. The guide body also includes structure associated with the slot for receiving the first cutter in the slot and preventing reception of the second cutter in the slot.

An advantage of the present invention is that a notch may be formed in the distal end of a femur, without the possibility of removing an excess amount of bone by using a milling cutter which is too large.

### BRIEF DESCRIPTION OF THE DRAWINGS

The above-mentioned and other features and advantages of this invention, and the manner of attaining them, will become more apparent and the invention will be better understood by reference to the following description of embodiments of the invention taken in conjunction with the accompanying drawings, wherein:
Fig. 1 is a top view of one embodiment of a milling guide of the present invention;
Fig. 2 is a side view of the embodiment shown in Fig. 1;
Fig. 3 is a side sectional view taken along line 3-3 of Fig. 1;
Fig. 4 is an elevational view taken along line 4-4 of Fig. 3;
Fig. 5 is a top view of another embodiment of a milling guide of the present invention;
Fig. 6 is a side view of the embodiment shown in Fig. 6;
Fig. 7 is a side sectional view taken along line 7-7 of Fig. 6; and
Fig. 8 is an elevational view taken along line 8-8 of Fig. 7.

Corresponding reference characters indicate corresponding parts throughout the several views. The exemplifications set out herein illustrate one preferred embodiment of the invention, in one form, and such exemplifications are not to be construed as limiting the scope of the invention in any manner.

### DETAILED DESCRIPTION OF THE INVENTION

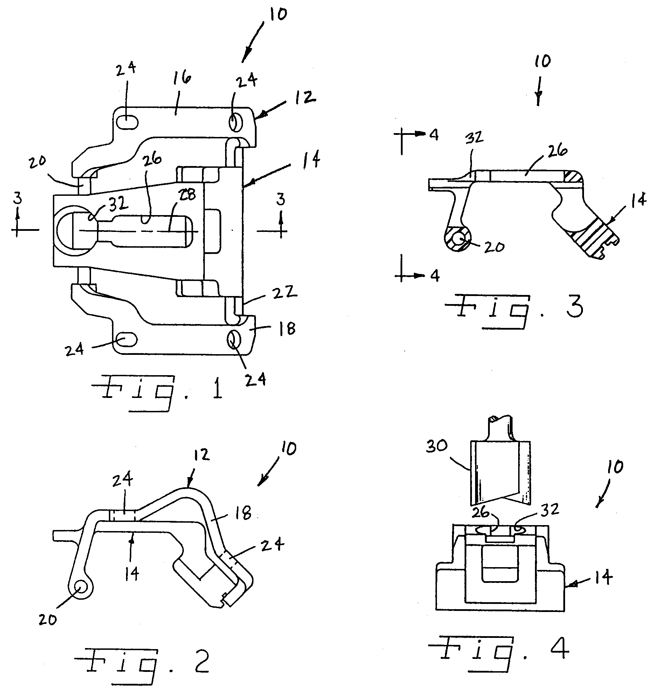

Referring now to the drawings and particularly to Figs. 1-4, an embodiment of a milling guide assembly 10 of the present invention is shown. In general, milling guide assembly 10 includes a frame 12 and guide body 14. Frame 12 has sides 16, 18 which are interconnected via bars 20, 22. Sides 16, 18 are each configured to matingly conform with bases (not shown) which are attached to the bone. Sides 16, 18 further include openings 24 for accommodating protuberances extending from the bases. For further details of frame 12, and bases to which frame 12 may be mounted, reference is made to the co-pending parent application, ,i.e., U.S. Patent Application Serial No. 08/169,459 (hereinafter referred to as the '459 application), which is hereby expressly incorporated herein by reference.

Guide body 14 is carried by bars 20, 22 and is shiftable on bars 20, 22 between sides 16, 18. Guide body 14 includes a slot 26 defining a longitudinal axis 28 thereof. Slot 26 is adapted to slidingly receive a milling cutter 30 (Fig. 4) therein. More particularly, and as described in the '459 application, slot 26 is configured to capture milling cutter 30 and inhibit movement of milling cutter 30 in directions other than in a direction corresponding to longitudinal axis 28.

Disposed generally co-planar and in communication with slot 26 is a circular opening 32. Opening 32 has a diameter which is slightly larger than a width/diameter of a predetermined corresponding milling cutter and allows passage of the milling cutter into the bone via a plunge cut.

Referring now to Fig. 4, a large milling cutter 30 is shown relative to opening 32. Guide body 14 is of a type which may be used to form a notch for a small femoral implant, such as a type "A" or "B" implant, and milling cutter 30 is of a type which may be used with a larger femoral implant. As is apparent, milling cutter 30 has a diameter which is larger than the diameter of opening 32. More particularly, in the embodiment shown in Figs. 1-4, milling cutter 30 has a diameter of about 3/4 inch and opening 32 has a diameter which is slightly larger than 1/2 inch. Thus, if it is attempted to pass milling cutter 30 through opening 32, the teeth of milling cutter 30 impinge on guide body 14 and prevent a plunge cut from being made into the bone. Excess removal of the bone is thereby prevented.

In operation, guide body 14 is attached to frame 12 and aligned relative to the distal end of the femur using anatomical landmarks. A milling cutter is then inserted through opening 32 and a plunge cut is made into the bone. Opening 32 is sized so as to only allow passage of a milling cutter having a width/diameter which is below a predetermined amount. Thus, for a size "A" or "B" femoral implant, opening 32 is sized so as to only allow a milling cutter with a diameter of about 1/2 inch or less to pass therethrough. The milling cutter is then moved in a direction along longitudinal axis 28 to form the notch in the distal end of the femur.

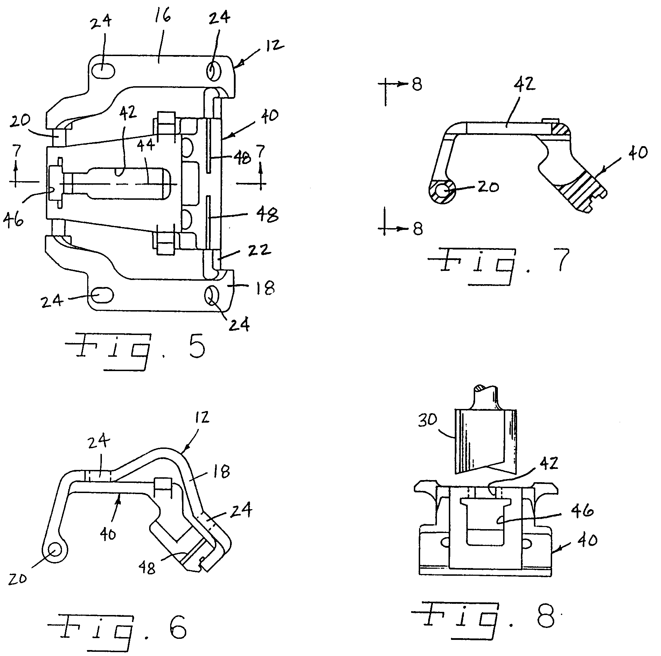

Referring now to Figs. 5-8, another embodiment of the present invention is shown. A guide body 40 is carried by a frame 12 as described above with regard to the embodiment shown in Figs. 1-4. Guide body 40 includes a slot 42 defining a longitudinal axis 44 thereof. Slot 42 is adapted to slidingly receive milling cutter 30 (Fig. 4) therein. Disposed generally orthogonal to and in communication with slot 42 is a rectangular opening 46. Opening 46 has a width which is slightly larger than a width/diameter of a predetermined corresponding milling cutter and allows passage of the milling cutter into the bone via a sideways cut. Conversely, opening 46 has a width which is less than a width/diameter of a milling cutter which is too large for the particular implant and prevents passage of such a milling cutter into the bone. Disposed at the top of opening 46 is an enlarged portion 48 which accommodates a collet of a powered rotatable driver (not shown) to which the milling cutter is attached. Guide body 40 also includes guide slots 48 for receiving and guiding a saw blade (not shown).

In operation, guide body 40 is attached to frame 12 and aligned relative to the distal end of the femur using anatomical landmarks. A milling cutter is then inserted through opening 46 and a sideways cut is made into the bone. Opening 46 is sized so as to only allow passage of a milling cutter having a width/diameter which is below a predetermined amount. Thus, for a size "A" or "B" femoral implant, opening 46 is sized so as to only allow a milling cutter with a diameter of about 1/2 inch or less to pass therethrough. The milling cutter is then moved in a direction along longitudinal axis 44 to form the notch in the distal end of the femur.

In the embodiments shown, slot 26 (Figs. 1-4) and slot 42 (Figs. 5-8) are single slots. However, it is to be understood that more than one slot could be formed in guide body 14. Such slots may or may not be in communication with each other.

Moreover, in the embodiments shown, the structures associated with the slot for receiving a first, smaller milling cutter in the slot and preventing a second, larger cutter from being received in the slot comprise a circular opening disposed generally co-planar with the slot and a rectangular opening disposed generally transverse to the slot. However, it is to be understood that differently shaped openings disposed either generally co-planar with or transverse to the slot, such as square, triangular, key-hole, etc., may be used. An important criterion is that if a milling cutter having a width/diameter above a predetermined amount cannot be used with a particular sized bone, then the guide body must be configured such that the milling cutter impinges upon the guide body prior to substantial damage being done to the bone.

While this invention has been described as having a preferred design, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A milling guide for use in orthopaedic surgery, for guiding one of a plurality of milling cutters, said plurality of cutters including a first cutter having a first diameter and a second cutter having a second diameter, said second diameter being larger than said first diameter, said milling guide comprising:
a guide body including a slot, said slot configured to slidingly receive the first cutter; and
means, associated with said slot, for receiving the first cutter in said slot and preventing reception of the second cutter in said slot.

2. The milling guide of Claim 1, wherein said receiving and preventing means comprises a circular opening formed in said guide body, said circular opening disposed in communication with said slot and said circular opening having a diameter which is larger than said first diameter and smaller than said second diameter.

3. The milling guide of Claim 2, wherein said circular opening is disposed substantially co-planar with said slot.

4. The milling guide of Claim 1, wherein said receiving and preventing means comprises an opening formed in said guide body, said opening disposed in communication with and transverse to said slot, said opening having a width which is larger than said first diameter and smaller than said second diameter.

5. The milling guide of Claim 4, wherein said opening is disposed in a non-planar relationship relative to said slot.

6. The milling guide of Claim 1, wherein said slot defines a longitudinal direction thereof, said slot configured to capture said cutter and inhibit movement of the cutter in directions other than said longitudinal direction.

7. The milling guide of Claim 1, wherein said first cutter has a diameter of approximately 1/2 inch, and said second cutter has a diameter of approximately 3/4 inch.

8. The milling guide of Claim 1, wherein said second cutter has a diameter which is larger than 1/2 inch.

9. The milling guide of Claim 1, wherein said slot comprises at least one slot.

10. The milling guide of Claim 1, wherein said slot extends to an edge of said guide body.

11. The milling guide of Claim 1, wherein said guide body further includes at least one guide slot for receiving and guiding a saw blade.

12. A method of guiding a milling cutter during an orthopaedic surgery, comprising the steps of:
locating a guide body relative to a bone to be milled, said guide body including a slot and an opening disposed in communication with said slot;
inserting one of a plurality of milling cutters into said opening;
plunge cutting said milling cutter through said opening and into the bone; and
sliding said milling cutter along said slot.

13. The method of Claim 12, wherein said opening is circular.

14. The method of Claim 12, wherein said opening prevents said plunge cutting step when said milling cutter is larger than a predetermined diameter.
